# EUROPEAN PATENT SPECIFICATION

(11) **EP 2 704 673 B1**
(45) Date of publication and mention of the grant of the patent: **07.10.2015**
(21) Application number: 12716989.4
(22) Date of filing: 17.04.2012
(51) Int. Cl.: A61M 25/10, A61F 7/12, A61B 18/14, A61B 18/00, A61B 18/02

(54) **COMPLIANT SLEEVES COUPLED WITH WIRE STRUCTURES FOR CRYOABLATION**
MIT DRAHTSTRUKTUREN GEKOPPELTE KONFORME MANSCHETTEN FÜR KRYOABLATION
MANCHONS SOUPLES AVEC STRUCTURES EN FIL MÉTALLIQUE POUR CRYO-ABLATION

(30) Priority: 02.05.2011 US 201113098551; 02.05.2011 US 201113098635; 02.05.2011 US 201113098709
(43) Date of publication of application: 12.03.2014
(73) Proprietor: Medtronic Cryocath LP, Toronto, Ontario M5L 1B9 (CA)
(72) Inventor: MIHALIK, Teresa Ann, Montreal Québec H3X 3P8 (CA); ZARBATANY, David, Laguna Niguel California 92677 (US); PATEL, Kaushik A., Poway California 92064 (US); ASCONEGUY, Alexander J., Murrieta California 92563 (US); LAY, Sitha, San Diego California 92115 (US); ROBISON, Karmi, Vista California 92081 (US)
(74) Representative: Casey, Lindsay Joseph
(86) International application number: PCT/US2012/033927
(87) International publication number: WO 2012/151048

(56) References cited:
- WO-A2-03/089997
- US-A1- 2002 072 708
- US-A1- 2003 060 813
- US-A1- 2009 048 668

## Description

### FIELD OF THE INVENTION

The present invention relates to medical systems for tissue diagnosis and treatment, and in particular to cardiac tissue mapping and ablation devices.

### BACKGROUND OF THE INVENTION

Minimally invasive devices, such as catheters, are often employed for surgical procedure, including those involving ablation, dilation, and the like. In a particular situation, an ablation procedure may involve creating a series of inter-connecting lesions in order to electrically isolate tissue believed to be the source of an arrhythmia. During the course of such a procedure, a physician may employ several different catheters having variations in the geometry and/or dimensions of the ablative element in order to produce the desired ablation pattern. Each catheter may have a unique geometry for creating a specific lesion pattern, with the multiple catheters being sequentially removed and replaced to create the desired multiple lesions. Each exchange represents an added risk to the patient as inserting and removing catheters in the vasculature carries a number of inherent risks, mainly embolism. Exchanging these various catheters during a procedure can cause inaccuracies or movement in the placement and location of the distal tip with respect to the tissue to be ablated, and may further add to the time required to perform the desired treatment. These potential inaccuracies and extended duration of the particular procedure increase the risk to the patient undergoing treatment.

Another factor adding to the complexity of minimally invasive techniques or procedures, such as cardiac mapping or ablation, is that treatment effectiveness and/or efficiency may rest largely on the ability to conformably position a medical device into contact with uneven or tortuous topography of a physiological structure or tissue region. For example, a treatment procedure may include thermal energy exchange with a targeted tissue site. Thus, not only is the thermal capacity of the medical device important, but the nature and extent of contact between the treatment region of the catheter and the adjacent tissue is important. Effective contact may require moving, positioning, anchoring and other mechanisms for positioning, stabilizing and changing the conformation of the treatment portion of the medical device. Slight changes in orientation may greatly alter the thermal range or characteristics of the medical device, so that even when the changes are predictable or measurable, it may become necessary to provide a high degree of conformability to assure adequate treatment at the designated sites. Aside from conformability for thermal transfer, some procedures include occluding a vessel or orifice, such as a pulmonary vein, to prevent extraneous thermal exchange with flowing blood or fluids around a medical device. Anatomical characteristics may vary widely from patient to patient, and so an extended range or capacity to selectively modify the shape or characteristics of a single medical device is highly desirable.

Such conformability is even more challenging to achieve when employing cryogenic cooling, such as in select electrophysiological mapping or ablation procedures. Many materials suffer substantial decreases in their elasticity or conformability when subjected to extremely low temperatures - i.e., the colder they get, the more rigid they become.

Accordingly, it would be desirable to provide a single medical device having the one or more treatment regions having an extended range of selectable shapes or dimensions, without the need for additional devices or the like having a single geometric orientation, and thus, limited in the ability to provide multiple ablative patterns. It is further desirable to provide a device that maintains high degrees of conformability at extremely low temperatures, such as those incurred during cryogenic ablation.

### SUMMARY OF THE INVENTION

According to an aspect of the present invention, there is provided a medical system as specified in Claim 1. According to another aspect of the present invention, there is provided a medical system as specified in any of claim 2 - 14.

The present invention advantageously provides systems having the one or more treatment regions with an extended range of selectable shapes or dimensions that maintain high degrees of conformability at extremely low temperatures.

In particular, a medical system is provided, including a catheter body, a deployable support structure coupled to the catheter body; an expandable element enclosing the support structure, the expandable element made from a compliant natural rubber emulsion; and a cryogenic coolant source in fluid communication with the expandable element. The natural rubber emulsion may include Yulex® HA. The support structure may include a mesh and/or a plurality of radially expandable struts, where at least one of the struts may define a fluid flow path therethrough. A distal portion of the expandable element may define the distal-most portion of the medical device. In an expanded state, the expandable element may define a substantially conical distal face and a substantially planar proximal face. The system may include a fluid injection lumen coupling the cryogenic coolant source to an interior of the expandable element, and a diameter of a distal portion of the fluid injection lumen may be selectively controllable. In an expanded state, the mesh may define a substantially conical distal face and a substantially planar proximal face. The system may include a radiofrequency signal generator in electrical communication with the mesh. The mesh may include at least one electrically-insulated portion and at least one electrically-conductive portion and/or may be controllably transitionable from a first shape to a second shape. The expansion of the expandable element may be inhibited at least in part by the mesh. The mesh may include a plurality of interwoven wires that are at least partially electrically-insulated, and the system may include a plurality of thermistors coupled to the mesh.

A cryogenic medical device is provided, including a flexible elongate body; a mesh coupled to a distal portion of the elongate body, the mesh selectively transitionable from a first geometric configuration to a second geometric configuration; and a compliant sleeve coupled to the mesh, the sleeve constructed from Yulex® HA. The mesh may be at least partially constructed from a shape-memory material; from at least one of Nitinol-Titanium alloy or stainless steel wire; from a textile or polymer; and/or may be biased towards the first geometric configuration.

A medical system is also provided, including a catheter body, an expandable element coupled to the catheter body; a first electrically-conductive element coupled to an interior surface of the expandable element; and a second electrically-conductive element coupled to an exterior surface of the expandable element, where the first and second electrically-conductive elements form a capacitor with the expandable element. The system may include a cryogenic coolant source in fluid communication with an interior of the expandable element; a fluid injection lumen coupling the cryogenic coolant source to an interior of the expandable element; and/or a support structure coupled to the expandable element, where the support structure may include a mesh or a plurality of radially expandable struts.

A medical system is also provided, including a flexible elongate body; an expandable element coupled to the elongate body; a first electrically-conductive element on an interior surface of the expandable element; a second electrically-conductive element on an exterior surface of the expandable element; and a control unit in electrical communication with the first and second electrically conductive elements, the control unit programmed to process capacitance measurements obtained from the first and second electrically conductive elements. The system may include a cryogenic coolant source in fluid communication with the elongate body; the control unit may be programmed to correlate a capacitance measurement to a contact force magnitude value; and/or at least one of the first or second electrically-conductive elements may include a layer of conductive ink adhered to the expandable element.

### BRIEF DESCRIPTION OF THE DRAWINGS

A more complete understanding of the present invention, and the attendant advantages and features thereof, will be more readily understood by reference to the following detailed description when considered in conjunction with the accompanying drawings wherein:
FIG. 1 is an illustration of an example of a medical system constructed in accordance with the principles of the present invention;
FIG. 2 is an illustration of an example of a distal region of a medical device of the system in FIG. 1;
FIG. 3 is another illustration of an example of a distal region of a medical device of the system in FIG. 1;
FIG. 4 is another illustration of an example of a distal region of a medical device of the system in FIG. 1;
FIGS. 5-11 illustrate examples of geometric configurations of the distal regions of FIGS. 1-4;
FIG. 12 is an illustration of an example of a distal region of a medical device of the system in FIG. 1;
FIG. 13 is an illustration of another example of a distal region of a medical device of the system in FIG. 1;
FIGS. 14-16 are additional illustrations of the distal region shown in FIG. 13;
FIGS. 17-19 illustrate exemplary methods of manufacturing a distal region of a medical device of the system in FIG. 1;
FIG. 20 is an illustration of an exemplary method of selectively adjusting a configuration of a medical device of the system in FIG. 1;
FIG. 21 is an illustration of an example of a sensor array for a medical device of the system in FIG. 1;
FIG. 22 is another illustration of an example of a sensor array for a medical device of the system in FIG. 1;
FIG. 23 is an illustration of an example of an assembly of a sensor of the array in FIGS. 21-22;
FIG. 24 is another illustration of an example of an assembly of a sensor of the array in FIGS. 21-22;
FIG. 25 is an illustration of an example of an electrical sensor mechanism for use with the system of FIG. 1;
FIG. 26 is an illustration of another example of an electrical sensor mechanism for use with the system of FIG. 1; and
FIG. 27 is an illustration of still another example of an electrical sensor mechanism for use with the system of FIG. 1.

### DETAILED DESCRIPTION OF THE INVENTION

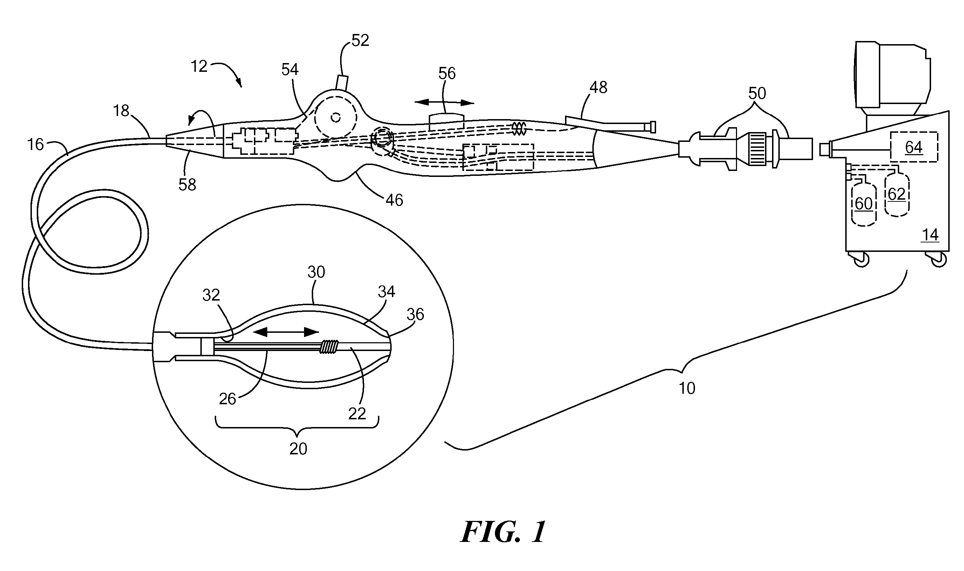

The present invention provides systems having the one or more treatment regions with an extended range of selectable shapes or dimensions that maintain high degrees of conformability at extremely low temperatures. Referring now to the drawing figures in which like reference designations refer to like elements, an embodiment of a medical system constructed in accordance with principles of the present invention is shown in FIG. 1 and generally designated as "10." The system 10 generally includes a medical device 12 that may be coupled to a control unit 14 or operating console. The medical device 12 may generally include one or more diagnostic or treatment regions for energetic, therapeutic and/or investigatory interaction between the medical device 12 and a treatment site. The treatment region(s) may deliver, for example, cryogenic therapy, radiofrequency energy, electroporation treatment or other energetic transfer with a tissue area in proximity to the treatment region(s), including cardiac tissue.

Referring to FIG. 1, the medical device 12 may include an elongate body 16 passable through a patient's vasculature and/or proximate to a tissue region for diagnosis or treatment, such as a catheter, sheath, or intravascular introducer. The elongate body 16 may define a proximal portion 18 and a distal portion 20, and may further include one or more lumens disposed within the elongate body 16 thereby providing mechanical, electrical, and/or fluid communication between the proximal portion of the elongate body 16 and the distal portion of the elongate body 16, as discussed in more detail below.

The medical device 12 may include a shaft 22 at least partially disposed within a portion of the elongate body 16. The shaft 22 may extend or otherwise protrude from a distal end of the elongate body 16, and may be movable with respect to the elongate body 16 in longitudinal and rotational directions. That is, the shaft 22 may be slidably and/or rotatably moveable with respect to the elongate body 16. The shaft 22 may further define a lumen therein for the introduction and passage of a guide wire and/or an auxiliary treatment or diagnostic instrument (not shown).

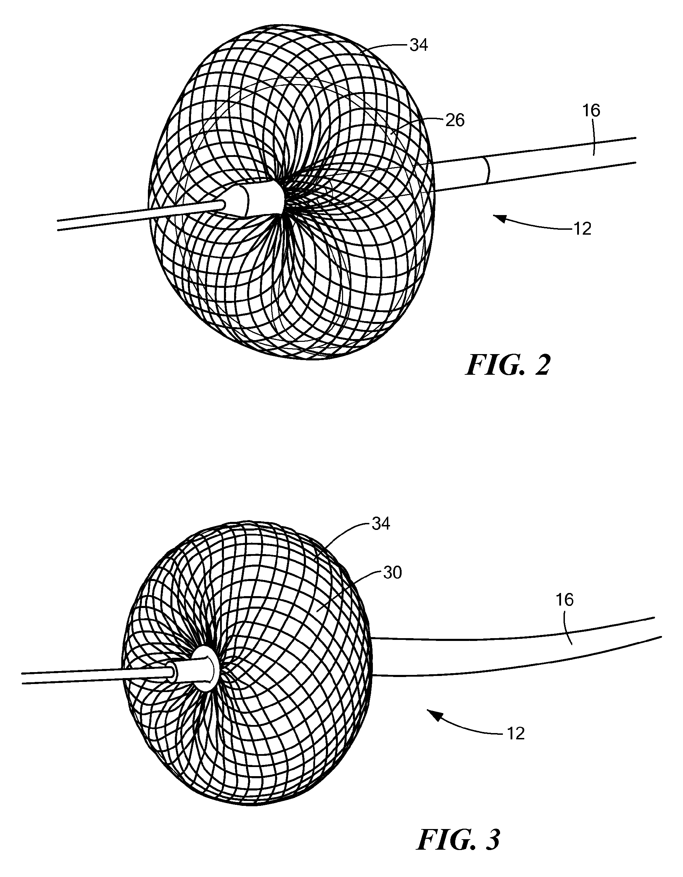

The medical device 12 may further include a fluid delivery conduit 26 traversing at least a portion of the elongate body 16 and towards the distal portion. The delivery conduit 26 may be coupled to or otherwise extend from the distal portion of the elongate body 16, and may further be coupled to the shaft 22 and/or distal tip of the medical device 12. For example, as shown in FIG. 1, the delivery conduit 26 may be helically coiled or otherwise wrapped around a portion of the shaft 22. Now referring to FIG. 2 (components of the medical device 12 are purposely omitted from FIG. 2 for ease of illustration), the delivery conduit 26 may be controllably expanded or otherwise directed outward from the shaft 22 and into closer proximity with one or more sections of an expandable/inflatable element or other distal components of the medical device 12, as described in more detail below, to provide direct fluid ejection and improved thermal effects. The fluid delivery conduit 26 may define a lumen therein for the passage or delivery of a fluid from the proximal portion of the elongate body 16 and/or the control unit 14 to the distal portion and/or treatment region of the medical device 12. The fluid delivery conduit 26 may further include one or more apertures or openings therein, to provide for the dispersion or directed ejection of fluid from the lumen to an environment exterior to the fluid delivery conduit 26. The fluid delivery conduit 26 may be coupled to one or more control or steering elements on a proximal portion of the medical device to selectively control a position, configuration, and/or shape of a distal portion of the delivery conduit 26.

The medical device 12 may further include one or more inflatable or expandable elements 30 at the distal portion of the elongate body 16. The expandable element 30 may be coupled to a portion of the elongate body 16 and also coupled to a portion of the shaft 22 to contain a portion of the fluid delivery conduit 26 therein. The expandable element 30 defines an interior chamber or region that contains coolant or fluid dispersed from the fluid delivery conduit 26, and may be in fluid communication with an exhaust lumen 32 defined by or included in the elongate body 16 for the removal of dispersed coolant from the interior of the expandable element 30. The expandable element 30 may provide a high degree of elasticity, compliance, or stretchability when subjected to cryogenic temperatures. For example, the ratio of an expanded diameter to an uninflated longitudinal length of the expandable element may be quite large, e.g., greater than 1. This expansion capability allows the expandable element 30 to have a shorter longitudinal length, which eases navigation in small tissue cavities or chambers, such as an atrium of the heart, while also allowing large expanded diameters to also ease occluding or otherwise contacting desired regions of tissue. In a particular example, the expandable element 30 may be constructed from a natural rubber emulsion such as Yulex® HA, which is surprisingly compliant at cryogenic temperatures. Unlike other rubber emulsions or polymers having limited compliance and increased rigidity at cryogenic temperatures, Yulex® HA maintains high elongation and modulus of elasticity characteristics at temperatures well below 0°C. The expandable element 30 may have any of a myriad of shapes, and may further include one or more material layers providing for puncture resistance, radiopacity, or the like.

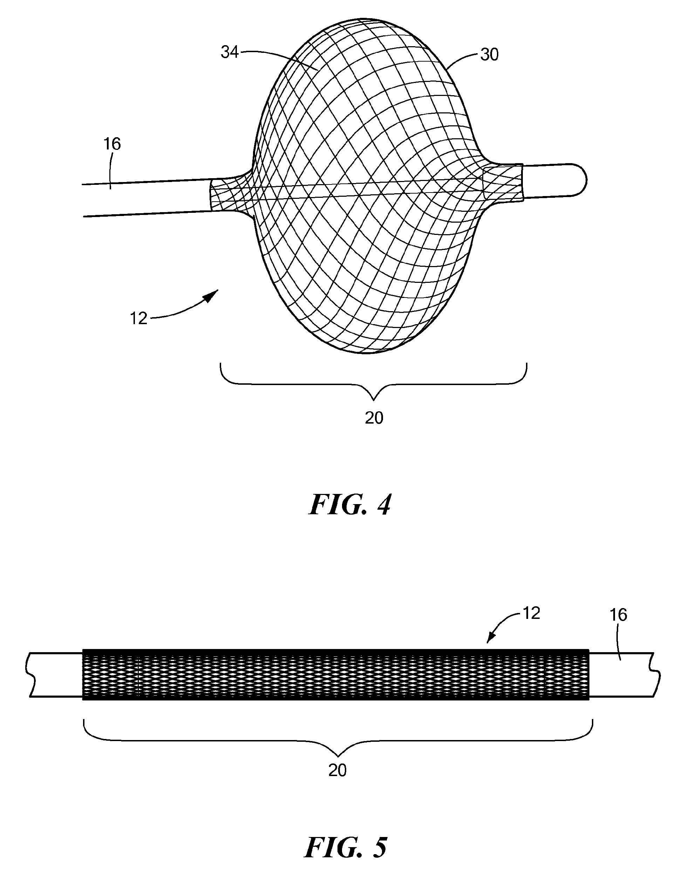

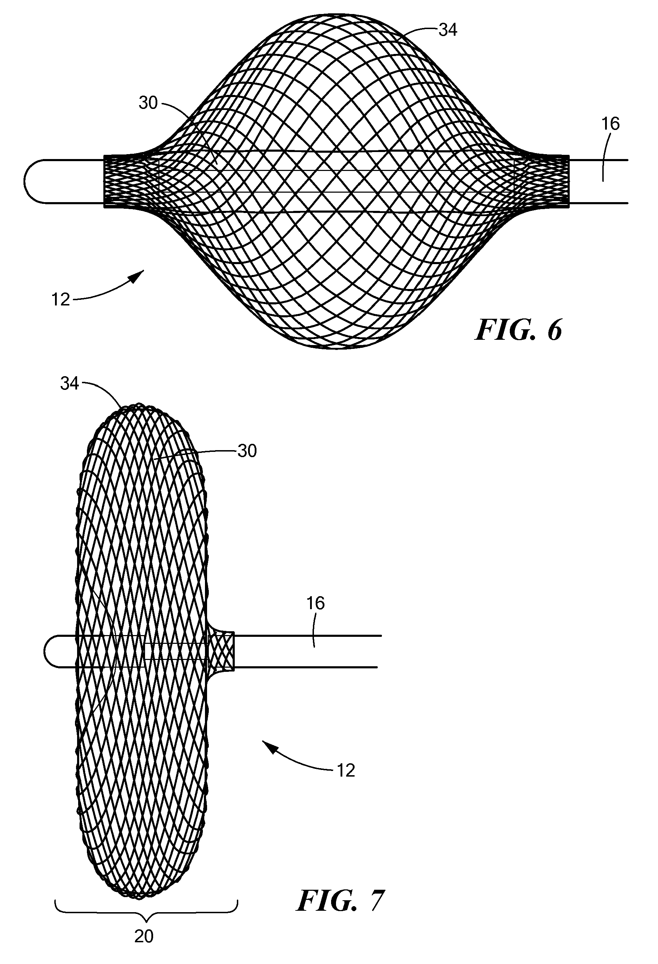

The medical device 12 may include a controllably deployable supporting structural element, frame, or scaffolding providing sufficient force to firmly contact a desired tissue region and/or facilitate a desired geometric configuration of the expandable element 30. For example, continuing to refer to FIGS. 1-4, the medical device 12 may include an expandable mesh 34 coupled to the distal portion of the elongate body 16. The mesh 34 may be configurable into a plurality of geometric configurations, such as those shown in FIGS. 5-11, for example. The mesh 34 may define an interwoven wire structure, and may be constructed from a combination of elastic materials, non-elastic materials, and/or shape-memory materials, such as a nickel-titanium alloy or the like, for example. The expandable mesh 34 can also be constructed of non-metallic materials, such as Nylon, Dacron, Kevlar or other fiber-type materials woven or otherwise set into the desired configuration. A particular geometric configuration of the mesh 34 may be achieved through the application of mechanical force, thermal energy, and/or electrical energy. For example, the mesh 34 may be predisposed and/or biased towards a first geometric configuration. Upon the application of a particular mechanical, thermal, and/or electrical force, the mesh 34 may be selectively transitioned from the first geometric configuration to a second geometric configuration.

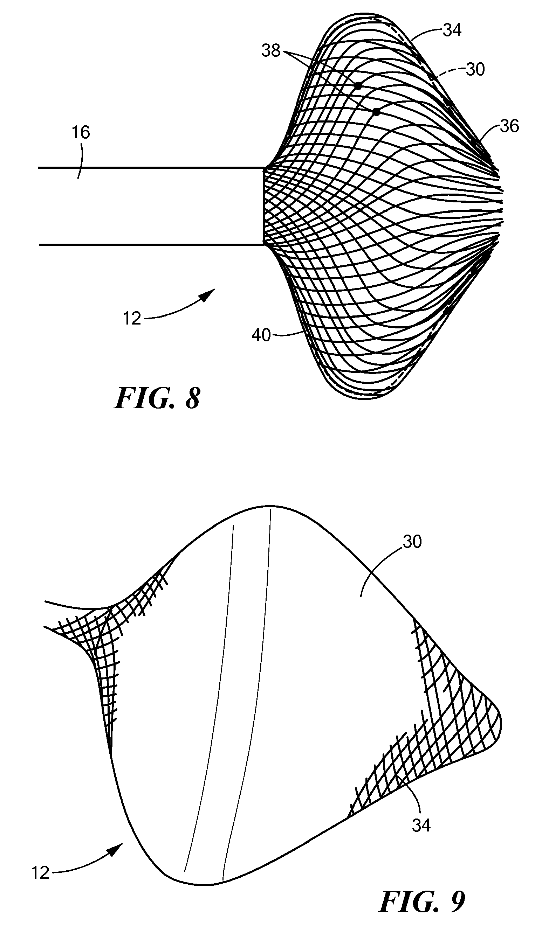

As shown in FIGS. 8-11, the mesh 34 may define a substantially continuous distal face or surface 36 that defines the distal-most point or contact region of the medical device 12. This is in contrast to prior art devices that have a rigid distal tip or protrusion at a distal end that prevents positioning a distal face or surface of a balloon or expandable element of the device against a substantially continuous tissue region, such as an atrial wall. With regards to the medical device 12, the absence of any such protruding, rigid distal tip or components allows the distal face 36 of the mesh 34 and the expandable element 30 to be placed directly against a tissue region without risking unintended injury to the tissue that a distal protrusion could otherwise inflict, and further allows enhanced contact across a wider area of tissue, resulting in better electrical and/or thermal communication than would otherwise be possible. The distal face 36 may include an opening allowing the exit of a guidewire or other instrument from the lumen in the shaft 22, but the opening may be substantially planar or contiguous with the portion of the mesh 34 and/or expandable element 30 immediately surrounding the opening such that the shaft 22 and/or any interfacing component, washer, or the like between the mesh 34, expandable element 30, and/or the shaft 22 has a minimal affect on the positioning of the distal face 36 of the mesh 34 against a tissue wall or region.

At least a portion of the mesh 34 may be electrically conductive to provide the ability to convey an electrical signal, current, or voltage to a designated tissue region and/or for measuring, recording, or otherwise assessing one or more electrical properties or characteristics of surrounding tissue. Portions of the mesh 34 may be electrically insulated, while other portions of the mesh 34 may be exposed and thus conductive of an electrical signal to facilitate contact and or use of the medical device 12 in targeted physiological areas. For example, conductive portions of the mesh 34 may be positioned at discrete locations about the expandable element 30, and may surround or encircle substantially all or only a fractional portion of the expandable members. Conductive portions of the mesh 34 may be asymmetrically disposed about the expandable member 30, e.g., positioned predominantly towards the proximal or distal portions of the expandable member 30, and/or on a side of the expandable member 30 likely to face a contacted tissue area.

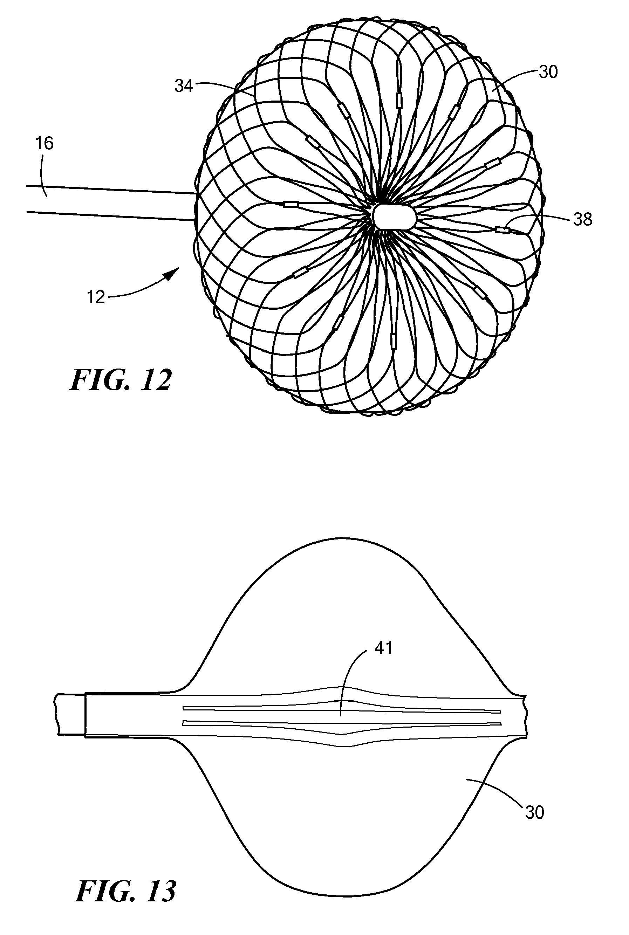

The exposed or otherwise electrically conductive portions of the mesh 34 may be present at one or more junctions 38 between the interwoven or intersecting wires that define the mesh 34, as shown in FIG. 12. The junctions 38 may present a plurality of conductive points or measurement locations on the medical device 12 for use in assessing or treating a targeted tissue area. For example, each junction 38 may be electrically coupled to an output portion of a radiofrequency or electrical signal generator (such as that described below), and each junction 38 may also include or define a sensor, such as a thermocouple/thermistor, an electrical conductivity sensor, a spectrometer, a pressure sensor, a fluid flow sensor, a pH sensor, and/or a thermal sensor (not shown) coupled to or in communication with the control unit 14 to trigger or actuate changes in operation when predetermined sequences, properties, or measurements are attained or exceeded.

The mesh 34 encloses the expandable element 30, as shown in FIG. 3. The mesh 34 may be immersed or coated in a material, such as Yulex® HA, to provide a sealed distal treatment region that is compliant or conformable to uneven tissue topography, while also providing selective, independent control over the geometric configuration of the medical device 12 through the mesh 34.

The mesh 34 and the expandable element or coating 30 are independently controlled or operated to provide the desired degree of conformability or compliance with an adjacent tissue structure. The mesh 34 may generally provide less compliant structure compared to the expandable element 30, such that the mesh 34 can impart its geometric characteristics or configuration onto the expandable element or coating 30 having increased elasticity, compliance, or stretchability. As such, irrespective of whether the expandable element 30 has a particular shape or dimensional capacity, the mesh 34 may be used to provide a guide and/or frame providing a desired geometric shape or configuration for at least a portion of the expandable element. The expandable element 30 may subsequently be inflated to a desired degree to achieve a desired geometric configuration across a remainder of the expandable element for optimal tissue coverage and/or contact.

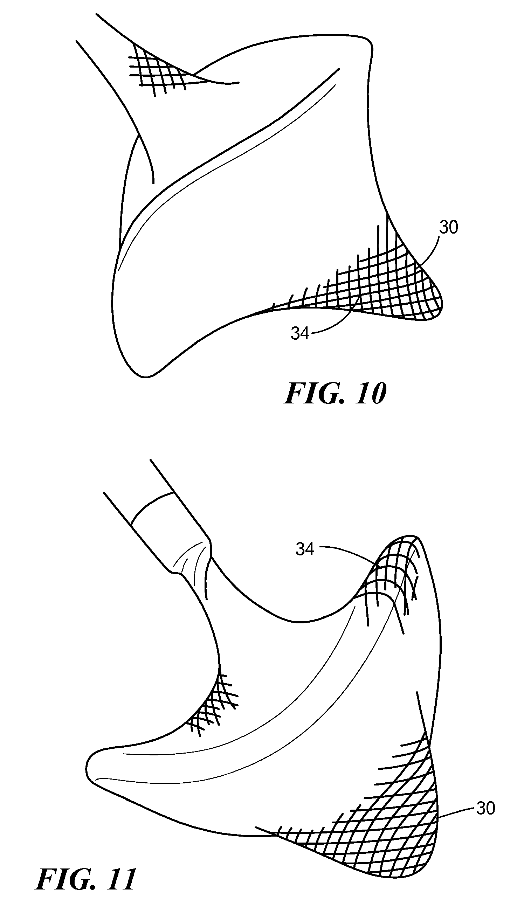

The mesh 34 may, accordingly, limit certain portions of the expandable element 30 from expanding or collapsing, while other areas or regions of the expandable element 30 may be controllably expanded or collapsed through manipulation of a circulating or delivered fluid to an interior of the mesh 34 and/or expandable element 30. For example, FIG. 9 shows a configuration where the expandable element 30 is inflated across substantially its entire length, while the mesh 34 is partially compressed to only marginally affect the shape of the expandable element 30. This configuration may be beneficial for occluding an orifice, such as a pulmonary vein opening or ostium. Turning to FIG. 10, the mesh 34 has been expanded radially and compressed longitudinally, coupled with a partial deflation of the expandable element 30. The resulting configuration includes a substantially planar proximal face 40 while providing a rounded, conical distal surface 36. This configuration may be beneficial for obstructing an orifice, or for conforming to a wide area of a tissue wall. FIG. 11 shows an alternative configuration where the expandable element 30 is mostly deflated while the mesh 34 provides an arcuate, disc-like shape. The distal portion of the expandable element provides a highly conformable or compliant reservoir tip that can be placed against a desired tissue region for thermal exchange, while sufficient contact force or torque can be applied through the mesh 34.

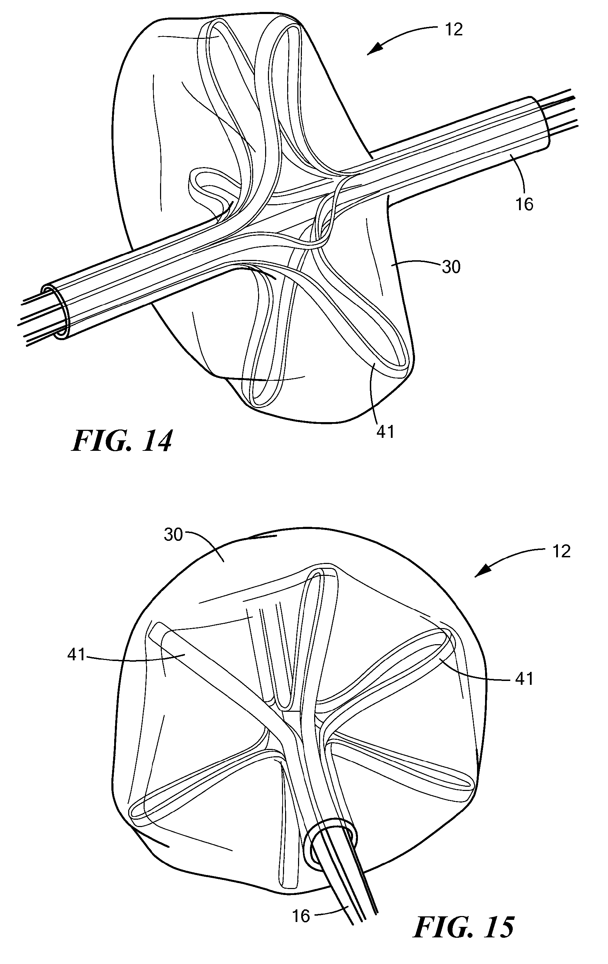

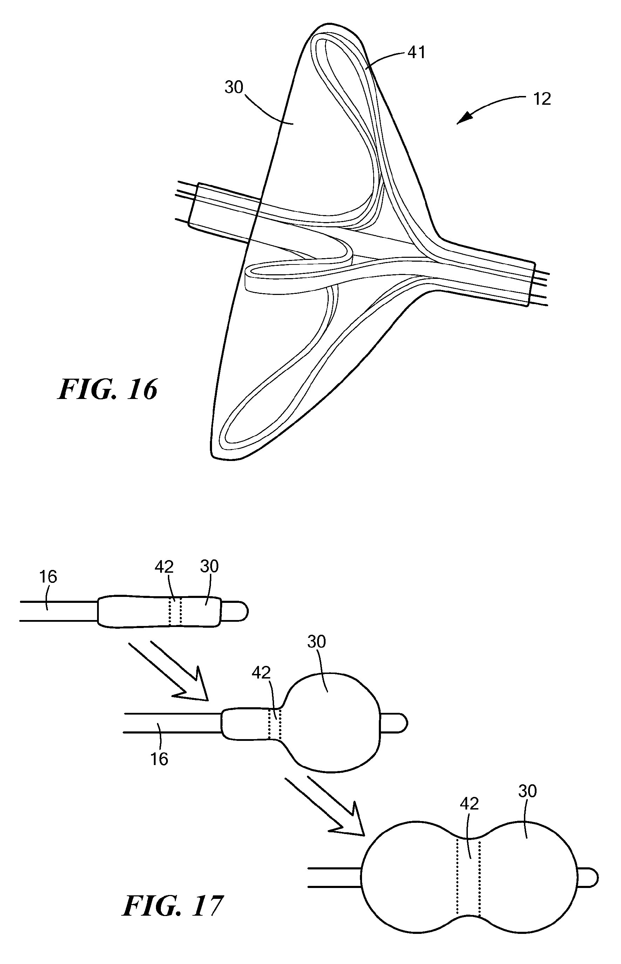

Now referring to FIGS. 13-16, the controllably deployable supporting structural element, frame, or scaffolding of the medical device 12 may include one or more struts 41 alternatively to the mesh 34. The struts 41 may be selectively deployable and retractable in a radial and/or longitudinal direction with respect to the elongate body 16 and/or the shaft 22 to achieve a desired geometric configuration of the distal region of the medical device 12. In addition, the struts 41 may be biased to present a first geometric configuration (such as an expanded state, for example), requiring an input force to overcome the biased configuration to achieve a secondary configuration (such as a retracted, minimally-transverse configuration). As shown in FIG. 13, the struts 41 may be retracted or otherwise positioned substantially parallel to the elongate body 16 and/or shaft 22, presenting a minimal transverse profile for ease of insertion and/or removal of the medical device. The struts 41 are independently operable of the inflation state or configuration of the expandable element 30. For example, as shown in FIGS. 14-15, the struts may be deployed radially outward from the shaft 22 to achieve a desired outer diameter, and the expandable element 30 may be partially inflated to present a pliable, conformable surface to a tissue region to be treated. As shown in FIG. 16, the struts 41 may be manipulated to present a "mushroom" shaped configuration having a substantially contoured, conical distal face and a planar or concave proximal face. Such a configuration may be suitable or desired to occlude an orifice or opening, such as within a pulmonary vein. The struts 41 may also include fluid apertures and/or flow paths therethrough to directly disperse fluid onto the expandable element 30 as an alternative to an independent fluid delivery conduit 26.

Of note, although a variety of geometric configurations are described above and shown in the accompanying figures, it is contemplated that a mesh 34 and/or struts 41 having more than two configurations may be employed and achieved through a combination of mechanical, thermal, and/or electrical forces, as well as through characteristics provided through material selection in the construction of the shaping element. Moreover, while examples and illustrations of particular geometric configurations have been provided, it is understood that virtually any shapes, configurations, and/or dimensions may be included and/or achieved by the medical device 12 of the present invention, including but not limited to those shapes illustrated and described herein. A particular geometric configuration may include circular, conical, concave, convex, rounded, or flattened features and/or combinations thereof. Accordingly, an embodiment of the medical device 12 of the present invention may be able to provide focal treatment patterns, wide area treatment patterns, circular treatment patterns, linear treatment patterns, circumferential treatment patterns, and combinations thereof.

The various geometric configurations of the mesh 34 and/or expandable element 30 may be achieved, at least partly, through a variety of manufacturing processes. For example, as shown in FIG. 17, a retaining structure 42 may be coupled to or integrated with the expandable element 30 to limit or otherwise affect expansion characteristics of the expandable element 30. The retaining structure may include, for example, an additional coating or layer of material, an annular ring, or the like, positioned in the region where the shape or expansion is to be adjusted. The retaining structure may be positioned longitudinally, radially, or in any configuration providing the desired expansion characteristics of the expandable element 30.

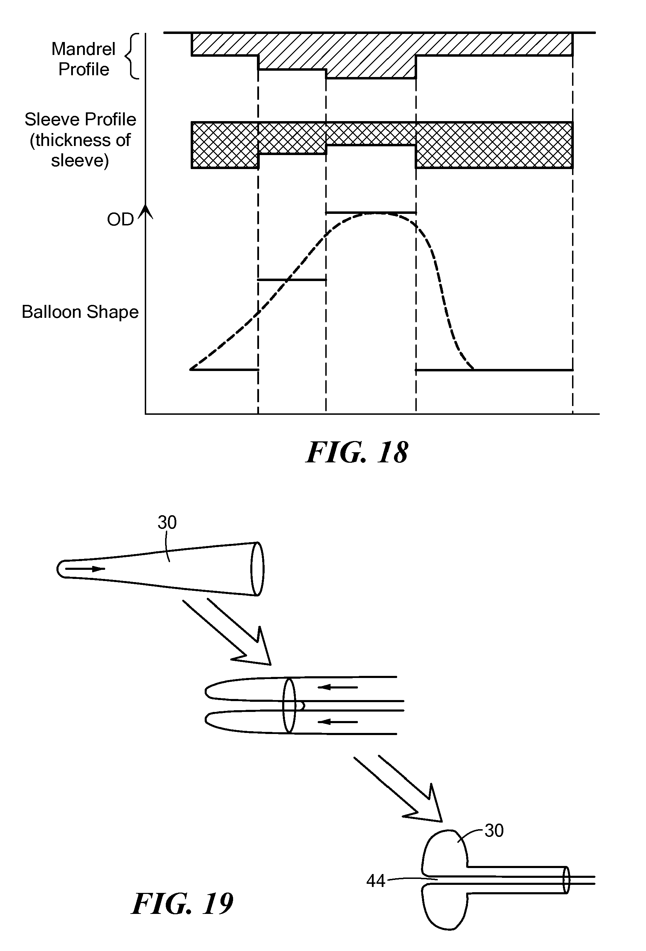

Now turning to FIG. 18, wall thickness characteristics may vary across one or more portions of the expandable element 30 to arrive at the desired expansion profile or shape. For example, a thickness of a mandrel or mold may vary across its length, resulting in mirrored variations in the material thickness along the expandable element 30. The varying thickness results in varied expansions, with thicker section having less expansion than thinner sections of the expandable element 30. Referring now to FIG. 19, the expandable element and one or more internal lumens 44, such as a guide wire lumen, may be formed by folding the expandable element back on itself, thereby creating a sealed distal end for circulating and/or delivering fluid.

Referring again to FIG. 1, the medical device 12 may include a handle 46 coupled to the proximal portion of the elongate body 16. The handle 46 can include circuitry for identification and/or use in controlling of the medical device 12 or another component of the system 10. Additionally, the handle 46 may be provided with a fitting 48 for receiving a guide wire or another diagnostic/treatment instrument. The handle 46 may also include connectors 50 that are matable to the control unit 14 to establish communication between the medical device 12 and one or more components or portions of the control unit 14.

The handle 46 may also include one or more actuation or control features that allow a user to control, deflect, steer, or otherwise manipulate a distal portion of the medical device 12 from the proximal portion of the medical device 12. For example, the handle 46 may include one or more components such as a lever or knob 52 for manipulating the elongate body 16 and/or additional components of the medical device 12. For example, a pull wire 54 with a proximal end and a distal end may have its distal end anchored to the elongate body 16 at or near the distal portion. The proximal end of the pull wire 54 may be anchored to an element such as a cam in communication with and responsive to the lever 52.

The medical device 12 may include one or more actuator elements 56 that are movably coupled to the proximal portion of the elongate body 16 and/or the handle 46 for the manipulation and movement of a portion of the medical device 12, such as the shaft 22, the fluid delivery conduit 26, the expandable element 30, and/or the mesh 34, for example. The actuator element(s) 56 may include a thumb-slide, a pushbutton, a rotating lever, or other mechanical structure for providing a movable coupling to the elongate body 16, the handle 46, and/or the shaft 22. Moreover, the actuator element 56 may be movably coupled to the handle 46 such that the actuator element 50 is movable into individual, distinct positions, and is able to be releasably secured in any one of the distinct positions. The medical device 12 may include one or more rotational control elements 58 that are rotatably coupled to the proximal portion of the fluid delivery conduit 26, shaft 22 and/or the handle 46 such that rotating the rotational control element 58 about a longitudinal axis of the handle 46 and/or elongate body 16 results in similar rotation of the shaft 22 and/or the fluid delivery conduit 26 at the distal portion of the medical device 12. The rotational control element 58 may include a knob, dial, or other mechanical structure for providing a rotatable coupling to the elongate body 16, the handle 46 and/or the shaft 22. Moreover, the rotational control element 58 may be rotatably coupled to the handle 46 and/or elongate body 16 such that the rotational control element 58 is movable into individual, distinct positions, and is able to be releasably secured in any one of the distinct positions.

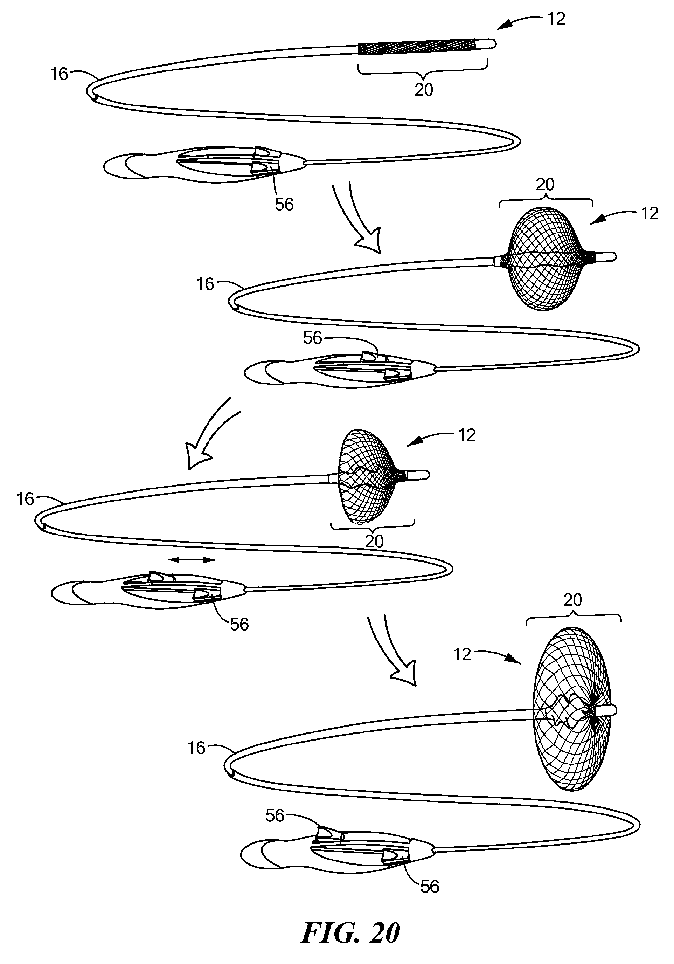

Manipulation of the actuator element(s) 56 and/or the rotational control element(s) 58 may provide movement of the fluid delivery conduit 26 to direct dispersed coolant or fluid flow onto a particular segment or region of the expandable element 30 for the desired clinical or therapeutic effect. In addition, the actuator element(s) 56 and/or rotational control element(s) 58 can be used to controllably position and/or rotate the shaft 22 of the medical device 12, the mesh 34, struts 41 and/or expandable element 30. For example, as shown in FIG. 20, the actuator elements 56 may be in a first position corresponding to or resulting in a substantially elongated, reduced radius profile of the distal portion 20 of the medical device 12. One of the actuator elements 56 may be manipulated in a first direction to expand the mesh 34 and/or struts 41 (not shown) independently of the expandable element 30. The actuator element may then be directed into a second position and/or second direction to substantially flatten or otherwise control a proximal face of the mesh 34. A second actuator element may also be manipulated to substantially flatten or otherwise control the shape of a distal face or surface of the mesh 34. Once the desired mesh configuration has been achieved, the expandable element 30 may be inflated to the desired degree, conforming to the selected shape of the mesh 34 and/or struts 41.

The system 10 may include one or more treatment or diagnostic sources coupled to the medical device 12 for use in an operative procedure, such as tissue ablation, for example. The control unit 14 may include a fluid supply 60 including a coolant, cryogenic refrigerant, or the like, an exhaust or scavenging system 10 (not shown) for recovering or venting expended fluid for re-use or disposal, as well as various control mechanisms. In addition to providing an exhaust function for the fluid or coolant supply, the control unit 14 may also include pumps, valves, controllers or the like to recover and/or re-circulate fluid delivered to the handle 46, the elongate body 16, and/or the fluid pathways of the medical device 12. A vacuum pump 62 in the control unit 14 may create a low-pressure environment in one or more conduits within the medical device 12 so that fluid is drawn into the conduit(s)/lumen(s) of the elongate body 16, away from the distal portion and towards the proximal portion of the elongate body 16.

The control unit 14 may include an electrical energy source 64 as a treatment or diagnostic mechanism in communication with one or more portions of the mesh 34 of the medical device 12. The electrical energy source 64 may include an electrical current or pulse generator, a radiofrequency generator or the like having a plurality of output channels, with each channel coupled to an individual junction. The electrical energy source 64 may be operable in one or more modes of operation, including for example: (i) bipolar energy delivery between at least two electrodes or electrically-conductive portions of the medical device 12 within a patient's body, (ii) monopolar or unipolar energy delivery to one or more of the electrodes or electrically-conductive portions on the medical device 12 within a patient's body and through a patient return or ground electrode (not shown) spaced apart from the electrodes of the medical device 12, such as on a patient's skin for example, and (iii) a combination of the monopolar and bipolar modes.

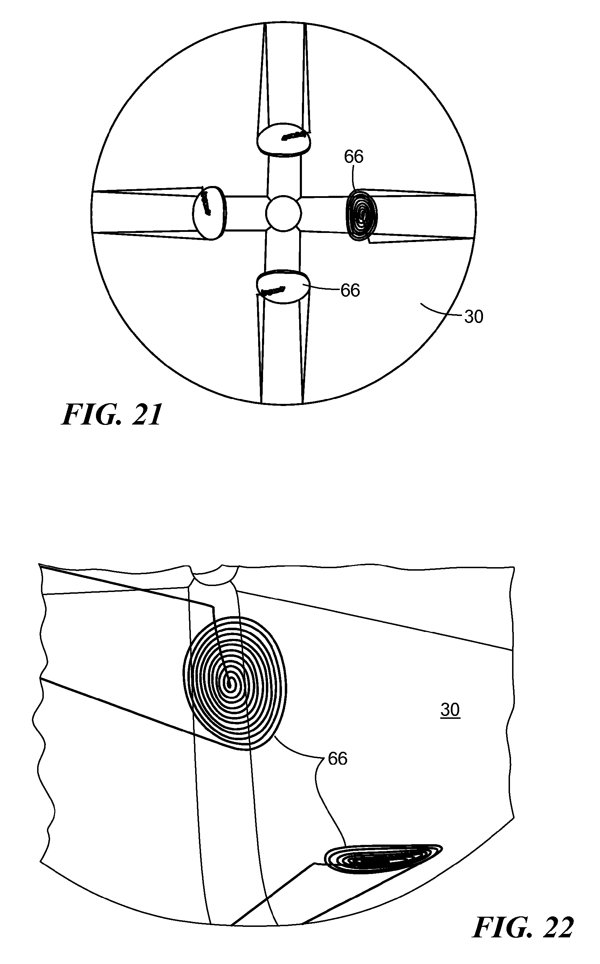

The system 10 may further include one or more sensors to monitor the operating parameters throughout the system 10, including for example, pressure, temperature, flow rates, volume, power delivery, impedance, or the like in the control unit 14 and/or the medical device 12, in addition to monitoring, recording or otherwise conveying measurements or conditions within the medical device 12 or the ambient environment at the distal portion of the medical device 12. Now referring to FIGS. 21-22, one or more sensors 66 may be coupled to the expandable element 30, mesh 34, and/or struts 41 that allow measurement or monitoring of one or more electrical properties and correlated conditions or status of the medical device 12 and the surrounding environment or contacted tissue. The sensors 66 may be radially positioned around the expandable element 30 to provide an indication of alignment or positioning of the expandable element based on differences or relationships between measured values obtained with the sensors 66.

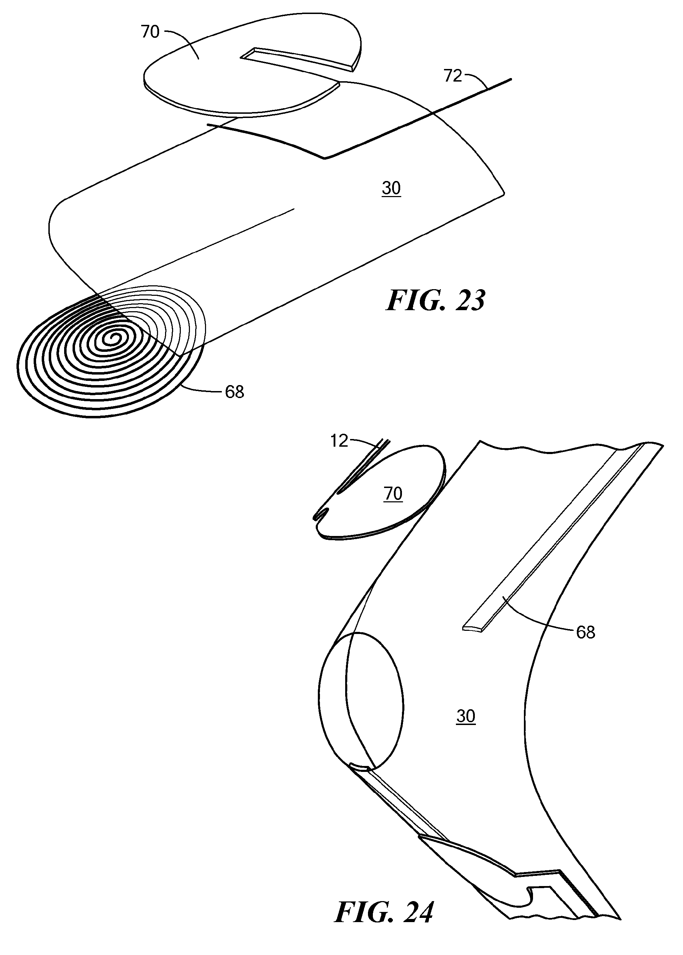

The sensors 66 may include one or more conductive ink layers deposited and cured on an elastomeric substrate layer (not shown) that is coupled to the expandable element 30, mesh 34, and/or struts 41. As an alternative method, a conductive ink or substrate may be applied directly onto the expandable element 30. In either configuration, the conductive ink may be placed on the expandable element 30 in predetermined geometries with alternating layers of conductive and non-conductive material in order to form a sensor. The use of an eleastomeric substrate allows the conductive layer to substantially match or conform to the stretching or expansion of the expandable element 30 as opposed to other sensor types that include rigid substrates. Turning now to FIGS. 23-24, one or more of the sensors 66 may include a first conductive element 68 positioned or adhered to an interior surface of the expandable element 30, while a second conductive element 70 is disposed on an exterior surface of the expandable element 30. A wire 72 may be coupled to the second conductive element 70 to transmit signals to and from the second conductive element 70 to and/or from the console 14. The expandable element 30 is disposed between the two conductive elements, presenting a dielectric medium to form a capacitor with the first and second conductive elements operable to relay electrical measurements and information (such as indications of tissue contact and/or electrical tissue activity, for example) to and from a proximal portion of the medical device 12 and/or the console 14. For example, a signal may be conducted through the wire 72 to the second conductive element 70, pass through the expandable element 30 and to the first conductive element 68, which provides a return path to the proximal end and/or console 14, where additional processing and/or calculations may be performed to correlate the measured signal to a tissue contact indication and/or an indication of electrical tissue activity.

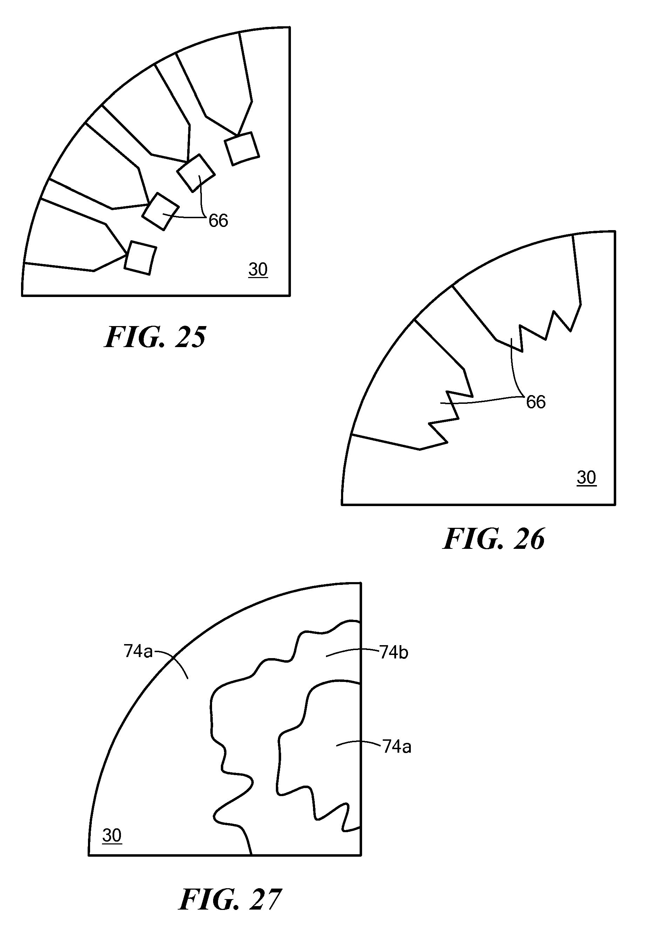

The sensors 66 may include a variety of different electrical property monitoring mechanisms. For example, as shown in FIG. 25, the sensors may include one or more voltage measuring mechanisms, while in FIG. 26, the sensors may operate to record or measure electrical resistance. Fig. 27 illustrated a plurality of conductive 74a and non-conductive layers 74b to provide a capacitance measuring mechanism similar to that shown in FIGS. 23-24.

The sensor(s) 66 and/or other sensors of the medical device 12 may be in communication with the control unit 14 for initiating or triggering one or more alerts or therapeutic delivery modifications during operation of the medical device 12. One or more valves, controllers, or the like may be in communication with the sensor(s) to provide for the controlled dispersion or circulation of fluid through the lumens/fluid paths of the medical device 12. Such valves, controllers, or the like may be located in a portion of the medical device 12 and/or in the control unit 14. The control unit 14 may include one or more controllers, processors, and/or software modules containing instructions or algorithms to provide for the automated operation and performance of the features, sequences, calculations, or procedures described herein.

In an exemplary use of the medical system 10, the distal portion 20 of the medical device 12 may be positioned in proximity to a tissue region to be treated. In particular, a portion of the mesh 34 and/or expandable element 30 may be positioned to contact a tissue region, such as a substantially continuous portion of an atrial wall, a circumference of a blood vessel, or the like. The mesh 34 and/or expandable element 30 may be manipulated into a desired geometric configuration. For example, the expandable element 30 may be inflated to a desired degree while the mesh 34 and/or struts 41 may be independently adjusted for the desired degree of radial and/or longitudinal expansion. Alternatively, the mesh 34 may be expanded or deployed to contact a tissue area while the expandable element 30 remains substantially uninflated.

The electrically-conductive portions of the mesh 34, such as the exposed or un-insulated junctions 38, and/or sensors 66 disposed on or otherwise coupled to the mesh, may be used to measure and/or record electrical properties or signals in the contacted tissue region. Such measuring or recording may include identifying aberrant electrical pathways in the tissue itself, commonly referred to as "mapping." The targeted tissue region may be mapped to identify the location of abnormal signal pathways for subsequent therapy or treatment. Further, regions of tissue identified or suspected of having such aberrant electrical activity may be temporarily electrically inhibited by reducing the temperature of the tissue. In particular, a coolant may be circulated through the expandable element 30, thus cooling tissue in proximity to the expandable element. The surrounding tissue may be cooled to a temperature that temporarily prevents or reduces electrical conduction without destroying or ablating the affected tissue - e.g., "cryo-mapping." Subsequent electrical measurement may be taken with the medical device 12 to confirm that the cryomapped segment should be treated further through the application of one or more ablative techniques.

Aside from mapping, the electrically-conductive portions of the mesh 34, such as the exposed or un-insulated junctions 38, and/or sensors 66 disposed on or otherwise coupled to the mesh, may be used to measure and/or record electrical properties or signals in the contacted tissue region to assess or otherwise generate an indication of a position, alignment, and/or occlusion of the targeted tissue region with the medical device 12. For example, the measured signals or properties may present an asymmetrical or skewed pattern of values with respect to a center or longitudinal axis of the mesh and/or medical device 12. This skewed or asymmetrical presentation may indicate that only a portion of the mesh 34 and/or struts 41 are in contact with the tissue and/or a tissue opening or orifice is not occluded or circumscribed by the mesh 34 and/or struts 41. Contact may also be assessed by changes in measured capacitance values. For example, the expandable element 30 may compress when the device contacts tissue, changing its dielectric characteristics between the first and second conductive elements 68, 70, and resulting in a rise time (an indication of the indirect capacitance value) for the measured parameter. The measured capacitance changes can then be correlated to a contact force magnitude through previously identified correlations/calibration techniques or calculations using the properties of the conductive elements 68, 70 and/or the expandable element 30. Accordingly, location and/or magnitude of contact between the device 12 and the tissue may be monitored or otherwise assessed with the sensors.

The system 10 may generate an indication based at least in part on the electrical measurements to inform the user whether the position, contact, and/or occlusion is sufficient to proceed with the designated procedure. The indication may include an audible signal and/or a visual indication (such as a green light, or a visual representation of the sensed pattern or location of the measured values with respect to the medical device or the tissue region). If the measured values correlate to a suitable position, the procedure may proceed. If the measured properties do not indicate a sufficient position or occlusion, the user may re-position the device and/or manipulate a geometric configuration of the mesh 34 and/or struts 41 and repeat the electrical property measurements.

Once attaining the desired position and/or confirmation that a tissue site is problematic, the medical device 12 may be used to treat the contacted tissue area. For example, the expandable element 30 of the medical device 12 is inflated separately and independently of the manipulation of the mesh 34 and/or struts 41. The expandable element 30 may, for example, be subjected to a fluid flow, including a cryogenic coolant or the like, to create an ablative lesion within a desired tissue region. The coolant may be controllably delivered through the fluid delivery conduit 26 and directed towards the expandable element 30 to obtain a desired temperature at the treatment site. A distal portion of the fluid delivery conduit may be selectively expanded or otherwise manipulated, via one or more controls on the handle for example, to place it into closer proximity to a desired sector or region of the expandable element 30, thereby improving thermal conduction or exchanged between a dispersed fluid and the expandable element and/or structural element, and thus the tissue.

In addition and/or alternatively to cryogenically treating the targeted tissue region, one or more portions of the mesh 34 may be used to conduct radiofrequency energy or electrical pulses into the tissue to create one or more ablation zones in the tissue. The radiofrequency energy may be delivered independently, simultaneously, and/or sequentially with the delivery of the cryogenic fluid flow through the expandable element 30 to achieve the desired clinical effect. Once a desired tissue region has been treated, the medical device 12 may be repositioned and/or reconfigured (i.e., the mesh 34, struts 41, and/or expandable element 30 may be reshaped) to create additional treatment regions having different geometric properties, resulting in the creation of a pattern of ablative lesions.

It will be appreciated by persons skilled in the art that the present invention is not limited to what has been particularly shown and described herein above. In addition, unless mention was made above to the contrary, it should be noted that all of the accompanying drawings are not to scale. Of note, the system components have been represented where appropriate by conventional symbols in the drawings, showing only those specific details that are pertinent to understanding the embodiments of the present invention so as not to obscure the disclosure with details that will be readily apparent to those of ordinary skill in the art having the benefit of the description herein. Moreover, while certain embodiments or figures described herein may illustrate features not expressly indicated on other figures or embodiments, it is understood that the features and components of the system and devices disclosed herein are not necessarily exclusive of each other and may be included in a variety of different combinations or configurations without departing from the scope of the invention. A variety of modifications and variations are possible in light of the above teachings without departing from the scope of the invention, which is limited only by the following claims.

## Claims

1. A medical system (10), comprising:
a catheter body (12),
an expandable element (30) made from a compliant natural rubber emulsion, and
a cryogenic coolant source in fluid communication with the expandable element (30);
**characterised by**:
a deployable support structure (34) coupled to the catheter body;
the deployable support structure enclosing the expandable element (30) and the expandable element (30) being independently controllable.

2. The medical system (10) of Claim 1, wherein the support structure (34) includes a mesh (34).

3. The medical system (10) of Claim 1, wherein the support structure (34) includes a plurality of radially expandable struts (41).

4. The medical system (10) of Claim 1, wherein a distal portion (20) of the expandable element (30) defines the distal-most portion of the medical device.

5. The medical system (10) of Claim 1, wherein in an expanded state, the expandable element (30) defines a substantially conical distal face (36) and a substantially planar proximal face (40).

6. The medical system (10) of Claim 1, further comprising a fluid injection lumen coupling the cryogenic coolant source (60) to an interior of the expandable element (30).

7. The medical system (10) of Claim 6, wherein a diameter of a distal portion (20) of the fluid injection lumen is selectively controllable.

8. The medical system (10) of Claim 2, wherein in an expanded state, the mesh (34) defines a substantially conical distal face (36) and a substantially planar proximal face.

9. The medical system (10) of Claim 2, further comprising a radiofrequency signal generator (64) in electrical communication with the mesh (34).

10. The medical system (10) of Claim 2, wherein the mesh (34) includes at least one electrically-insulated portion and at least one electrically-conductive portion.

11. The medical system (10) of Claim 2, wherein the mesh (34) is controllably transitionable from a first shape to a second shape.

12. The medical system (10) of claim 11, wherein the expansion of the expandable element (30) is inhibited at least in part by the mesh (34).

13. The medical system (10) of Claim 2, wherein the mesh (34) includes a plurality of interwoven wires that are at least partially electrically-insulated.

14. The medical system (10) of Claim 2, further comprising a plurality of thermistors coupled to the mesh (34).

## Patentansprüche

1. Medizinische Vorrichtung (10), die Folgendes umfasst:
einen Katheterkörper (12),
ein expandierbares Element (30) aus einer nachgiebigen Kautschukemulsion; und
eine Quelle von kryogenem Kühlmittel in Fluidverbindung mit dem expandierbaren Element (30);
**gekennzeichnet durch**
eine entfaltbare Stützstruktur (34), die an den Katheterkörper gekoppelt ist;
wobei die entfaltbare Stützstruktur (34) das expandierbare Element (30) umschließt und das expandierbare Element (30) unabhängig steuerbar ist.

2. Medizinisches System (10) nach Anspruch 1, wobei die Stützstruktur (34) ein Netz (34) umfasst.

3. Medizinisches System (10) nach Anspruch 1, wobei die Stützstruktur (34) mehrere radial expandierbare Streben (41) umfasst.

4. Medizinisches System (10) nach Anspruch 1, wobei ein distaler Abschnitt (20) des expandierbaren Elements (30) den am weitesten distal gelegenen Abschnitt der medizinischen Vorrichtung definiert.

5. Medizinisches System (10) nach Anspruch 1, wobei in einem expandierten Zustand das expandierbare Element (30) eine im Wesentlichen konische distale Fläche (36) und eine im Wesentlichen ebene proximale Fläche (40) definiert.

6. Medizinisches System (10) nach Anspruch 1, weiter umfassend ein Fluidinjektionslumen, das die Quelle (60) von kryogenem Kühlmittel an ein Inneres des expandierbaren Elements (30) koppelt.

7. Medizinisches System (10) nach Anspruch 6, wobei ein Durchmesser eines distalen Abschnitts (20) des Fluidinjektionslumens selektiv steuerbar ist.

8. Medizinisches System (10) nach Anspruch 2, wobei in einem expandierten Zustand das Netz (34) eine im Wesentlichen konische distale Fläche (36) und eine im Wesentlichen ebene proximale Fläche definiert.

9. Medizinisches System (10) nach Anspruch 2, weiter umfassend einen Funkfrequenz-Signalgenerator (64) in elektrischer Verbindung mit dem Netz (34).

10. Medizinisches System (10) nach Anspruch 2, wobei das Netz (34) mindestens einen elektrisch isolierten Abschnitt und mindestens einen elektrisch leitfähigen Abschnitt umfasst.

11. Medizinisches System (10) nach Anspruch 2, wobei das Netz (34) steuerbar von einer ersten Form zu einer zweiten Form überführt werden kann.

12. Medizinisches System (10) nach Anspruch 11, wobei die Expansion des expandierbaren Elements (30) mindestens teilweise von dem Netz (34) gehemmt wird.

13. Medizinisches System (10) nach Anspruch 2, wobei das Netz (34) mehrere miteinander verwobene Drähte umfasst, die mindestens teilweise elektrisch isoliert sind.

14. Medizinisches System (10) nach Anspruch 2, weiter umfassend mehrere an das Netz (34) gekoppelte Thermistoren.

## Revendications

1. Système médical (10), comprenant :
un corps de cathéter (12),
un élément extensible (30) réalisé en une émulsion de caoutchouc naturel souple ; et
une source de réfrigérant cryogénique en communication fluidique avec l'élément extensible (30) ;
**caractérisé par**
une structure de support déployable (34) couplée au corps de cathéter ;
la structure de support déployable (34) enfermant l'élément extensible (30) et l'élément extensible (30) pouvant être commandé indépendamment.

2. Système médical (10) selon la revendication 1, dans lequel la structure de support (34) comporte un filet (34).

3. Système médical (10) selon la revendication 1, dans lequel la structure de support (34) comporte une pluralité d'entretoises extensibles radialement (41).

4. Système médical (10) selon la revendication 1, dans lequel une partie distale (20) de l'élément extensible (30) définit la partie la plus distale du dispositif médical.

5. Système médical (10) selon la revendication 1, dans lequel dans un état étendu, l'élément extensible (30) définit une face distale sensiblement conique (36) et une face proximale sensiblement plane (40).

6. Système médical (10) selon la revendication 1, comprenant en outre une lumière d'injection de fluide qui couple la source de réfrigérant cryogénique (60) à un intérieur de l'élément extensible (30).

7. Système médical (10) selon la revendication 6, dans lequel un diamètre d'une partie distale (20) de la lumière d'injection de fluide est sélectivement contrôlable.

8. Système médical (10) selon la revendication 2, dans lequel dans un état étendu, le filet (34) définit une face distale sensiblement conique (36) et une face proximale sensiblement plane.

9. Système médical (10) selon la revendication 2, comprenant en outre un générateur de signaux radiofréquence (64) en communication électrique avec le filet (34).

10. Système médical (10) selon la revendication 2, dans lequel le filet (34) comporte au moins une partie électriquement isolée et au moins une partie électriquement conductrice.

11. Système médical (10) selon la revendication 2, dans lequel le filet (34) est modifiable de façon contrôlée d'une première forme à un seconde forme.

12. Système médical (10) selon la revendication 11, dans lequel l'extension de l'élément extensible (30) est empêchée au moins en partie par le filet (34).

13. Système médical (10) selon la revendication 2, dans lequel le filet (34) comporte une pluralité de fils métalliques entrelacés qui sont au moins partiellement isolés électriquement.

14. Système médical (10) selon la revendication 2, comprenant en outre une pluralité de thermistances couplées au filet (34).
